# EUROPEAN PATENT APPLICATION

(11) **EP 1 657 250 A1**
(43) Date of publication of application: **17.05.2006**
(21) Application number: 04026827.8
(22) Date of filing: 11.11.2004
(51) Int. Cl.: C07K 14/47, A61K 39/00, C07K 7/04, G01N 33/574, C12Q 1/68

(54) **HLA-A *01-binding T-cell epitope of WT1**

(71) Applicant: Charité - Universitätsmedizin Berlin, 10117 Berlin (DE)
(72) Inventor: Scheibenbogen, Carmen, 12203 Berlin (DE); Asemissen, Anne Marie, 10829 Berlin (DE); Stevanovic, Stefan, 72076 Tübingen (DE); Keilholz, Ulrich, 12203 Berlin (DE)
(74) Representative: Engelhard, Markus

(57) **Abstract**

The present invention refers to an immunogenic HLA-A*01-binding T-cell epitope of the Wilms' tumour 1 protein (WT1) and its utility as a target in immunotherapy. In more detail, the invention refers to a polypeptide comprising an amino acid sequence as depicted in SEQ ID NO: 1, to an antibody directed to said polypeptide, to a polynucleotide encoding said amino acid sequence and to a polynucleotide which is complementary to said polynucleotide. The invention further refers to pharmaceutical compositions and *in vitro* applications.

## Description

Briefly, the present invention refers to an immunogenic HLA-A*01-binding T-cell epitope of the Wilms' tumour 1 protein (WT1) and its utility as a target in immunotherapy. In more detail, the invention refers to a polypeptide comprising an amino acid sequence of said HLA-A*01-binding T-cell epitope or a portion or variant thereof.

### Background of the invention

Wilms' tumour (WT) is an embryonal malignancy of the kidney affecting infants and young children. The development of WT has been linked to the inactivation of a tumour suppressor gene both by epidemiologic studies and by genetic analyses. The observation of karyotype abnormalities in predisposed children and studies of the molecular genetics of WT tissue specimens enabled the identification of chromosome band 11 p 13 as one genetic locus on the human chromosome inactivated in WT. Finally, a gene in 11p13, the so-called wt1 gene, was isolated and characterized.

A protein product of the wt1 gene, the zinc finger transcription factor WT1 (Swissprot Accession No. P 19544), is overexpressed in leukaemias and various types of solid tumours suggesting an important role of WT1 in both leukaemogenesis and tumourigenesis. Accordingly, WT 1 has attracted much interest as a target for cancer therapy. More recent approaches have focused on immunotherapeutic strategies based on the generation or enhancement of an immune response to tumour-associated antigens (TAAs) such as WT1.

Several HLA class 1-binding WT1 epitopes for HLA-A*0201 and HLA-A*24 have been identified (e.g. ref. 3-5). Although most studies on WT1 T-cell epitopes refer to HLA class I molecules, particularly to HLA-A, there are also some reports referring to HLA class II-restricted T-cell responses (e.g. ref. 31).

An early evidence that human cytotoxic T-lymphocytes (CTLs) specifically respond to WT1 peptides containing HLA-A2.1-binding anchor motifs was provided by Oka *et al.* (4). Different groups have succeeded in generating HLA-A2-restricted anti-leukemic CTLs specific for WT1-derived peptides. A CD8⁺ CTL clone was established, designated TAK-1, which clone is specific for a WT1-derived 9-mer peptide consisting of HLA-A24-binding anchor motifs (28). Adoptive transfer of TAK-1 into nude mice engrafted with a HLA-A24-positive lung cancer cell line resulted in inhibition of cancer cell growth and prolonged survival (29). In C57BL/6 mice it was shown that subjects immunized with a WT1 peptide or WT1 cDNA rejected challenges by WT1-expressing tumour cells and survived with no signs of auto-aggression to WT1-expressing normal organs by the induced CTLs (30).

A first clinical support for the promising concept of WT1 peptide-based immunotherapy was provided by Oka *et al.* (32). In patients with overt leukaemia from myelodysplastic syndrome (MDS) or MDS with myelofibrosis only a single dose of WT1 vaccination using an HLA-A*2402-restricted 9-mer WT1 peptide resulted in an increase in WT1-specific CTLs, which was followed by a rapid reduction in leukemic blast cells. Further results from phase I clinical studies were reported by the same group (33, 34). Another clinical trial showed that vaccination of patients with the HLA-A*0201-binding WT1 epitope WT1.126-134 induced high frequency T-cell responses which were associated with complete remission in a patient with relapsed acute myeloid leukaemia (AML) (2).

The Corixa Corporation (US) is applicant of two families of patent applications derived from WO 00/18795 (PCT/US99/22819) and WO 01/25273 (PCT/US00/27465), both referring to compositions and methods for WT1 specific immunotherapy.

WO 00/18795 discloses polypeptides comprising an immunogenic portion of a native WT1 or a variant thereof that differs in one or more substitutions, deletions, additions and/or insertions such that the ability of the variant to react with antigen-specific antisera and/or T-cell lines or clones is not substantially diminished. Within certain embodiments, the polypeptide comprises no more than 16 consecutive amino acid residues of a native WT1 polypeptide. Within other embodiments, the polypeptide comprises an immunogenic portion of amino acid residues 1-174 of a native WT1 polypeptide or a variant thereof, wherein the polypeptide comprises no more than 16 consecutive amino acid residues present within amino acids 175 to 449 of the native WT polypeptide. The immunogenic portion preferably binds to an MHC class I and/or class II molecule. The invention further provides methods for enhancing or inducing an immune response in a patient as well as methods for inhibiting the development of a malignant disease in a patient, comprising administering to a patient a pharmaceutical composition or vaccine comprising the polypeptides disclosed. Malignant diseases according to the invention include leukaemias (e.g. acute myeloid, acute lymphocytic and chronic myeloid) and cancers (e.g. breast, lung, thyroid or gastrointestinal cancer or a melanoma).

A prerequisite for successful immunotherapy is the knowledge of immunogenic T-cell epitopes. The search for new T-cell epitopes in known TAAs using the classical "reverse immunology" strategy has led to the identification of several T-cell epitopes, most of them restricted to HLA-A*02 (6). This strategy includes the prediction of potential T-cell epitopes from known tumour antigens, their analysis for MHC-binding, followed by *the in vitro* generation of peptide-reactive T-cells and their testing of target cell recognition. This strategy is rather laborious because of the need for T-cell induction against multiple peptides, which are often not processed. However, recognition of TAAs through T-cells requires processing in an antigen presenting cell und presentation together with a HLA molecule. In the setting of non-HLA-A*0201 alleles target tumour cells expressing both the respective HLA allele and the target antigen are often not readily available.

A more recent approach to increase the efficacy of identifying potential T-cell epitopes is the *ex vivo* screening of candidate epitopes for recognition by T-cells from patients which are naturally primed against the target antigen (7-10). Candidate epitopes can be predicted using appropriate prediction *models in silico* (e.g. ref. 17). WO 00/18795 used the BIMAS HLA peptide binding prediction analysis (35). An elegant approach to show epitope processing is by *in vitro* proteasome-mediated digestion pattern analysis. This approach has reliably identified HLA-A*02-binding epitopes from tumour and viral proteins using 25-30-mer peptides encompassing the putative epitope (11,12).

Even though such *in silico* prediction models as mentioned above may be highly efficacious to predict candidate epitopes, the necessity remains to verify their utility *in vitro* and *in vivo.* Thus, there is still a need to identify those epitopes having superior properties.

Therefore, it is an object of the present invention to provide epitopes of particular interest for use in immunotherapy and in the analysis or natural T-cell immunity.

### Summary of the invention

The object of the present invention is solved by a polypeptide comprising an amino acid sequence as depicted in SEQ ID NO: 1 or a portion or a variant thereof.

The object of the present invention is solved by a polypeptide consisting of an amino acid sequence as depicted in SEQ ID NO: 1 or a portion or a variant thereof.

In one embodiment, the polypeptide is an immunogenic HLA-A*01-binding T-cell epitope of a WT1 protein.

In one embodiment, the polypeptide is for use as a pharmaceutical.

The object of the present invention is further solved by a composition comprising the polypeptide according to the invention.

In one embodiment, the composition is for use as a pharmaceutical.

The object of the present invention is further solved by a pharmaceutical composition comprising the polypeptide according to the invention.

In one embodiment, the pharmaceutical composition additionally comprises at least one polypeptide comprising an amino acid sequence or a portion or a variant thereof selected from the group comprising amino acid sequences as depicted in SEQ ID NO: 8-20.

The object of the present invention is further solved by a use of the polypeptide of the present invention for the manufacture of a pharmaceutical for the treatment of a disease selected from the group comprising WT1 expressing leukaemia and solid tumours.

In one embodiment, the leukaemia is acute myeloid leukaemia.

The object of the present invention is further solved by an *in vitro* use of the polypeptide according to the invention.

In one embodiment, the polypeptide is used for stimulating and/or expanding T cells in a sample.

In one embodiment, the sample is selected from the group comprising bodily fluid and cultured T-cells.

The object of the present invention is further solved by a method for preparing the polypeptide according to the invention.

The object of the present invention is further solved by a diagnostic kit comprising the polypeptide according to the invention.

The object of the present invention is further solved by an antibody recognizing the polypeptide according to the invention.

The object of the present invention is further solved by a composition comprising the antibody according to the present invention.

In one embodiment, the composition is for use as a pharmaceutical.

The object of the present invention is further solved by a use of the antibody according to the present invention for the manufacture of a pharmaceutical for the treatment of a disease selected from the group comprising WT1 expressing leukaemia and solid tumours.

In one embodiment, the leukaemia is acute myeloid leukaemia.

The object of the present invention is further solved by an *in vitro* use of the antibody according to the invention.

In one embodiment, *the in vitro* use is for identification and/or selection of one or more cells expressing the polypeptide of the present invention.

In one embodiment, *the in vitro* use is for diagnosis and/or monitoring of a disease selected from the group comprising WT 1 expressing leukaemia and solid tumours.

In one embodiment, the leukaemia is acute myeloid leukaemia.

The object of the present invention is further solved by a method for diagnosis and/or monitoring of a disease selected from the group comprising WT1 expressing leukaemia and solid tumours, preferably acute myeloid leukaemia, using the antibody according to the invention

In one embodiment, the method comprises the steps of:
(a) providing a sample obtained from a patient suspected to contain the polypeptide according to the invention;
(b) contacting said sample with the antibody according to the invention;
(c) detecting and/or quantifying an interaction between the polypeptide of (a) and the antibody of (b);
(d) comparing the result obtained in (c) with a reference and/or with a result obtained from a reference sample.

The object of the present invention is further solved by a method for preparing the antibody according to the invention.

The object of the present invention is further solved by a diagnostic kit comprising the antibody according to the invention.

The object of the present invention is further solved by a polynucleotide encoding an amino acid sequence as depicted in SEQ ID NO: 1 or a portion or a variant thereof.

In one embodiment, the polynucleotide is for use as a pharmaceutical.

The object of the present invention is further solved by a composition comprising the polynucleotide according to the invention.

In one embodiment, the composition is for use as a pharmaceutical.

The object of the present invention is further solved by a use of the polynucleotide according to the invention for the manufacture of a pharmaceutical for the treatment of a disease selected from the group comprising WT1 expressing leukaemia and solid tumours.

In one embodiment, the leukaemia is acute myeloid leukaemia.

The object of the present invention is further solved by an *in vitro* use of the polynucleotide according to the invention.

The object of the present invention is further solved by a diagnostic kit comprising the polynucleotide according to the invention.

The object of the present invention is further solved by an antibody recognizing the polynucleotide according to the invention.

The object of the present invention is further solved by a composition comprising the antibody recognizing the polynucleotide according to the invention.

In one embodiment, the composition is for use as a pharmaceutical.

The object of the present invention if further solved by a use of the antibody recognizing the polynucleotide according to the invention for the manufacture of a pharmaceutical for the treatment of a disease selected from the group comprising WT1 expressing leukaemia and solid tumours.

In one embodiment, the leukaemia is acute myeloid leukaemia.

The object of the present invention is further solved by an *in vitro* use of the antibody recognizing the polynucleotide according to the invention.

In one embodiment, *the in vitro* use is for identification and/or selection of one or more cells producing the polynucleotide according to the invention.

In one embodiment, the *in vitro* use is for diagnosis and/or monitoring of a disease selected from the group comprising WT1 expressing leukaemia and solid tumours.

In one embodiment, the leukaemia is acute myeloid leukaemia.

The object of the present invention is further solved by a method for diagnosis and/or monitoring of a disease selected from the group comprising WT1 expressing leukaemia and solid tumours, particularly acute myeloid leukaemia, using the antibody recognizing the polynucleotide according to invention.

In one embodiment, the method comprises the steps of:
(a) providing a sample obtained from a patient suspected to contain a polynucleotide according to the invention;
(b) contacting said sample with the antibody recognizing the polynucleotide according to the invention;
(c) detecting and/or quantifying an interaction between the polynucleotide of (a) and the antibody of (b);
(d) comparing the result obtained in (c) with a reference and/or with a result obtained from a reference sample.

The object of the present invention is further solved by a method for preparing the antibody recognizing the polynucleotide according the invention.

The object of the present invention is further solved by a diagnostic kit comprising an antibody recognizing the polynucleotide according to the invention.

The object of the present invention is further solved by a polynucleotide complementary to the polynucleotide according to the invention.

The object of the present invention is further solved by a composition comprising the polynucleotide complementary to the polynucleotide according to the invention.

In one embodiment, the composition is for use as a pharmaceutical.

The object of the present invention is solved by a use of the polynucleotide complementary to the polynucleotide according to the invention for the manufacture of a pharmaceutical for the treatment of a disease selected from the group comprising WT1 expressing leukaemia and solid tumours.

In one embodiment, the leukaemia is acute myeloid leukaemia.

The object of the present invention is further solved by an *in vitro* use of the polynucleotide complementary to the polynucleotide according to the invention.

In one embodiment, the *in vitro* is for identification and/or selection of one or more cells producing the polynucleotide according to the invention.

In one embodiment, *the in vitro* use is for diagnosis and/or monitoring of a disease selected from the group comprising leukaemia and solid tumours.

In one embodiment, the leukaemia is acute myeloid leukaemia.

The object of the present invention is further solved by a method for diagnosis and/or monitoring of a disease selected from the group comprising WT1 expressing leukaemia and solid tumours, particularly acute myeloid leukaemia, using the polynucleotide complementary to the polynucleotide according to the invention.

In one embodiment, the method comprises the steps of:
(a) providing a sample obtained from a patient suspected to contain the polynucleotide according to the invention;
(b) contacting said sample with the polynucleotide recognizing the polypeptide according to the invention;
(c) detecting and/or quantifying an interaction between the polynucleotide of (a) and the antibody of (b);
(d) comparing the result obtained in (c) with a reference and/or with a result obtained from a reference sample.

The object of the present invention is further solved by a method for preparing the polynucleotide recognizing the polynucleotide according to the invention.

The object of the present invention is further solved by a kit comprising the polynucleotide recognizing the polynucleotide according to the invention.

The object of the present invention is further solved by a cytotoxic T-lymphocyte recognizing a polypeptide according to the invention.

The object of the present invention is further solved by an antigen presenting cell expressing a polypeptide according to the invention.

The object of the present invention is solved by a method of identification of an immunogenic T-cell epitope comprising the following steps:
(a) prediction of a potential epitope
(b) screening of said epitope for recognition by T-cells; and
(c) *in vitro* degradation proteasomal cleavage.

In one embodiment, the T-cell epitope is one of a WT protein.

The object of the present invention is further solved by a method of treatment using a polypeptide as depicted in SEQ ID NO:1.

In one embodiment, the method of treatment additionally comprises the use of at least one polypeptide comprising an amino acid sequence or a portion or a variant thereof selected from the group comprising amino acid sequences as depicted in SEQ ID NO: 8-20.

The object of the present invention is further solved by a method of treatment using a polynucleotide encoding the amino acid sequence as depicted in SEQ OD NO: 1 or a portion or a variant thereof.

In one embodiment, the method of treatment additionally comprises at least one polynucleotide encoding an amino acid sequence or a portion or a variant thereof selected from the group comprising amino acid sequences as depicted in SEQ ID NO: 8-20.

A "variant" of an amino acid according to the present invention differs in one or more substitutions, deletions, additions, and/or insertions.

The term "immunogenic" as used herein refers to the capability of an agent, i.e. an antigen, to induce a specific immune reaction of an organism, i.e. production of antibodies or immunocompetent lymphocytes, the latter are grouped into B- and T-cells.

The term "HLA-A*01" refers to a subgroup of HLA ("human leukocyte antigen") class I molecules on the basis of the HLA system.

The term "T-cell epitope" refers to a portion of an antigen, also referred to as antigenic determinant, which is recognised and bound by T-cells. The T-cell epitope according to the present invention refers to a portion of a WT1 protein.

A "WT1 protein" according to the present invention comprises natural WT1 proteins of any species, preferably of mammalian species, most preferably of human. A natural WT1 protein is a naturally occurring protein. A natural WT1 protein also may be naturally mutated. The present invention further considers WT1 proteins that differ from natural WT1 proteins due to manipulations.

A polypeptide according to the present invention is immunogenic and reacts with WT1-specific antisera and/or T-cell lines or clones.

A "WT1 expressing leukaemia" comprises WT1 expressing AML and CML. Amongst patients suffering from AML or CML, such patients showing a complete remission after allogeneic transplantation are preferred.

A "composition for use a pharmaceutical" comprises compositions for administration routes selected from the group of parenteral (e.g. *i.v., i.d., s.c., i.m., i.p),* oral, nasal, anal, vaginal and topical administration.

A dosage form of a pharmaceutical composition according to the present invention is selected from the group comprising solid dosage forms (e.g. tablets, pills, capsules, suppositories, powders), liquid dosage forms (e.g. infusions, elixirs, syrups), further dosage forms (e.g. gels, sprays, ointments) and also slow release dosage forms. In a preferred embodiment, the dosage form is a sterile liquid for vaccination via i.m. bolus injection.

The pharmaceutical compositions according to the present invention can be prepared using one or more pharmaceutically acceptable carrier or excipients and according to common compounding techniques. In a preferred embodiment, the excipient is a non-specific immune response enhacer, i.e. an adjuvant. In a particularly preferred embodiment, the adjuvant is keyhole limped hemocyanin (KLH) in combination with GM-CSF.

A pharmaceutical composition according to the present invention may contain at least one polypeptide comprising an amino acid sequence or a portion or a variant thereof selected from the group comprising the amino acid sequences as depicted in SEQ ID NO: 8-20 in the absence of a polypeptide comprising or consisting of an amino acid sequence or a portion or a variant thereof as depicted in SEQ ID NO: 1.

A "cytotoxic T-lymphocyte" recognising a polypeptide according to the present invention comprises a lymphocyte obtained from bodily fluids and tissues, a cell in primary cell culture and a cell of an immortal cell line.

Examples for proteasomal cleavage *in vitro* comprises the use of constitutive and immunoproteasomes. Such proteasomal cleavage assays are used in order to demonstrate natural processing of a polypeptide. The term "natural processing" refers to a process running in a natural eukaryotic cell after termination of the synthesis of a polypeptide on the ribosome. This process comprises cleavage of the polypeptide to form a mature protein or peptide, modification of amino acid residues, folding of the polypeptide into its active three-dimensional conformation, and targeting of the polypeptide for degradation.

### Brief description of the figures

- **Fig. 1**: shows the flow cytometric analysis of T-cell responses to HLA-A*01-binding candidate epitopes from WT1 as depicted in SEQ ID NO: 1-5 in 14 patients with haematologic malignancies.
- **Fig. 1A**: shows the frequencies of specific CD3⁺CD8⁺ T-cells secreting IFNγ in response to the 5 WT1 peptides as mentioned above and an irrelevant HLA-A1- binding peptide (GSEELRSLY, from HIV gag p17 70-78) as depicted in SEQ ID NO: 7.
- **Fig.1B**: shows a representative example of the T-cell response analysis against WT1.317-327 as depicted in SEQ ID NO: 1 in one HLA-A*01-positive patient detected by intracellular IFNγ and staining with an A*01/WT1.317-327 tetramer. The CD8/IFNγ profile of CD3 gated lymphocytes in PBMC in response to the HLA-A*01-binding HIV peptide *(left),* and to WT1.317-327 *(middle)* is shown. The *right* diagram shows the tetramer staining of CD3⁺ gated T-cells.
- **Fig. 2**: shows the proteasome-mediated digestion of the precursor peptide WT1.314-336 as depicted in SEQ ID NO: 6.
- **Fig. 3**: shows candidate T-cell epitopes of WT1 for HLA-A1, HLA-B7 and HLA-B44 as depicted in SEQ ID NO: 8-20.

### Detailed description of the invention

In this study, we have evaluated a strategy to directly identify immunogenic T-cell epitopes from WT1 combining the following steps: 1) Prediction of potential epitopes by the SYFPEITHI algorithm, 2) screening of potential epitopes for recognition by T-cells from tumour patients with haematological malignancies, and 3) *in vitro* degradation by constitutive and immunoproteasomes to demonstrate natural epitope processing. We focussed on HLA-A*01 because of its relatively high prevalance (15%) among the Caucasian population and the high predictive value of the prediction algorithm used for this allele (7).

Successful predictions of HLA-A*01-restricted epitopes with the algorithm developed by Rammensee's group have been carried out previously (7,8). The current study further supports the validity of this prediction model for HLA-A*01-binding epitopes. Our finding also supports the applicability of the Prediction Algorithm for Proteasomal Cleavage (PAProC, 24). Using PAProC, the epitope WT1.317-327 was identified in this study and its sequence is depicted in SEQ ID NO: 1. WT1.317-327 is predicted to be generated by constitutive as well as immune proteasomes, while the C-terminus of the other 4 peptides (SEQ ID NO: 2-5) is not predicted to be generated by either proteasome.

Taken together the strategy selected for epitope identification in this study was demonstrated to be a rapid and reliable approach to identify an immunogenic T-cell epitope from the tumour antigen WT1. Such epitopes are of great interest for analysing the natural immunity in tumour patients and for application in antigen-specific immunotherapy.

### EXAMPLE 1: Identification of WT1.317-327

### Patients and healthy controls

PBMC from HLA-A*01-positive patients and healthy subjects were collected and cryopre-served. The investigation had been approved by the Institutional Ethics Committee and informed consent was obtained from all individuals.

### Predicition of HLA-A*01-binding peptides

Prediction of candidate 9-mer and 10-mer epitopes according to the HLA-A*01 motif from WT1 (swissprot Accession No. P19544) was performed using the SYFPEITHI algorithm and 11 to 13-mer candidate epitopes were calculated according to the same prediction model as described (17). Using this approach, five peptides were selected as candidate T-cell epitopes including one 11-mer (WT1.317-327; SEQ ID NO: 1), two 12-mer (WT1.37-48; SEQ ID NO: 2; and WT1.260-271; SEQ ID NO: 3), and two 13-mer (WT1.137-149; SEQ ID NO: 4; and WT1.188-200; SEQ ID NO: 5) peptides.

### Peptide synthesis

Peptides were synthesized in an Applied Biosystems 432A peptide synthesizer following standard protocols. Synthesized products were analysed by high-performance liquid chromatography (Varian Star; Zinsser Analytics, Munich, Germany) and MALDI-TOF mass spectrometry (G2025A; Hewlett-Packard, Waldbronn, Germany), and purified by preparative HPLC to purities >95%.

### T-cell analysis

Antigen-specific T-cells were detected by two methods. In a functional assay intracellular IFNγ accumulation induced by WT1 peptide was assessed by flow cytometry as described previously (13). For tetramer analyses PE-labelled HLA-A*01 WT1.317-327 (SEQ ID NO: 1) tetrameric complexes were synthesized essentially as described (14). In order to reduce background by avoiding unspecific binding to CDS, tetramers with the mutation A245V in the HLA alpha chain were used (15).

### Proteasomal processing

Purification of 20S proteasomes, *in vitro* degradation of WT1.314-336 (SEQ ID NO: 6), and separation and analysis of cleavage products were performed as described (16). 10 nmol (27.6 µg) of WT1.314-336 were incubated for 6 h with 2 µg immunoproteasome or with 2 µg of constitutive proteasome, respectively, in digestion buffer (20 mM Tris-HCI; pH 7.6; 10 mM NaCl; 10 mM KCI; 2 mM MgCl₂; 0.5 mM DTT) and the reaction stopped by freezing the reaction mixture at -80°C.

### Screening of T-cell reactivity to predicted epitopes in AML patients and allogeneic stem cell recipients

Using functional flow cytometry detecting peptide-induced induction of intracellular IFNγ we analyzed circulating T-cells from patients for recognition of the 5 WT1 candidate epitopes (SEQ ID NO: 1-5). HLA-A*01-positive patients from whom PBMC samples were available for this analysis included 4 AML patients and 8 patients who had received allogeneic stem cell transplantation (SCT) for AML, lymphoma or myeloma. Increased frequencies of CD8⁺ T-cells specific for various leukaemia-associated antigens including WT1 have been reported in patients with myeloid leukaemias after allogeneic SCT (18-20). Patients with lymphoma and myeloma were included in this analysis since expression of WT1 was also found in lymphoma and plasmocytoma cell lines. Further 2 HLA-A*26 patients were included, since HLA-A*26 had been grouped into the HLA-A*01 family (21).

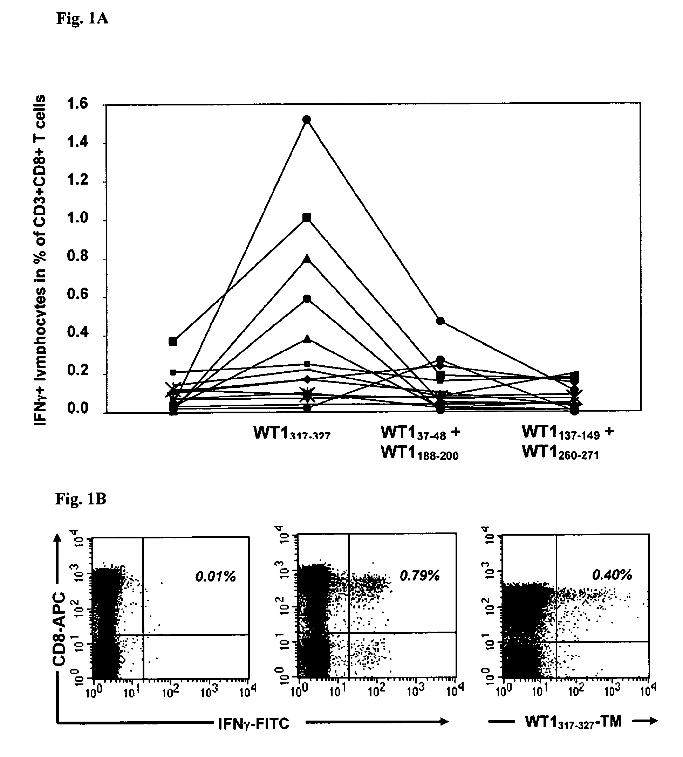

Flow cytometric analysis of T-cell responses to HLA-A*01-binding candidate epitopes from WT1 in 14 patients with hematologic malignancies is shown in **Fig. 1**. Analysis of PBMC was performed by three-color flow cytometry. Frequencies of specific CD3⁺CD8⁺ T-cells secreting IFNγ in response to the 5 WT1 peptides and an irrelevant HLA-Al-binding peptide from HIV gag p17 70-78 (SEQ ID NO: 7) are shown in **Fig.1A.** The T-cell response against both WT1.37-48 (SEQ ID NO: 2) and WT1.188-200 (SEQ ID NO: 5), and WT1.137-149 (SEQ ID NO: 4) and WT1.260-271 (SEQ ID NO: 3), respectively, were analyzed in one sample. **Fig. 1B** shows a representative example of the T-cell response analysis against WT1.317-327 (SEQ ID NO: 1) in one HLA-A*01-positive patient detected by intracellular IFNγ and staining with an A*01/WT1.317-327 tetramer. The CD8/IFNγ profile of CD3 gated lymphocytes in PBMC in response to the HLA-A*01-binding HIV peptide *(left),* and to WT1.317-327 *(middle)* is shown. The right diagram shows the tetramer staining of CD3⁺ gated T-cells.

As demonstrated in **Fig. 1**, the candidate epitope WT1.317-327 (SEQ ID NO: 1) showed a remarkably high immunogenicity as T-cells specifically secreting IFNγ in response to this peptide were detected in 2 of 4 AML patients, and in 3 of 10 SC recipients with frequencies of 1.5%, 0.8%, 0.6%, 0.6% and 0.4% of CD3⁺CD8⁺ T-cells, respectively (**Fig. 1A** and **B**, percentage of IFNγ+ CD3⁺CD8⁺ T-cells in response to HIV peptide subtracted). In contrast, no T-cell response to this epitope was detected in 10 HLA-A*01-positive healthy subjects. The natural immunogenicity of WT1.317-327 is comparable to that of the HLA-A*02-binding epitope WT.126-134 (13).

### In vitro proteasome digestion of WT1.314-337

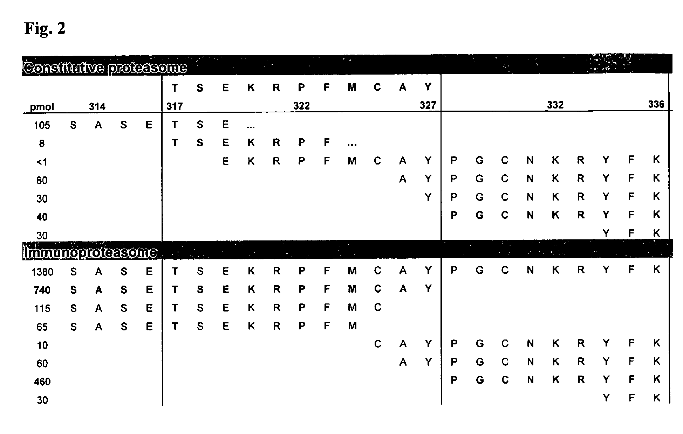

One crucial prerequisite for a peptide to be presented as a T-cell epitope is its proper excision from the protein. We analyzed *in vitro* digestion of the 24mer WT1-derived peptide 314-336 (SEQ ID NO: 6) encompassing the WT1.317-327 epitope TSEKRPFMCAY (SEQ ID NO: 1) by constitutive as well as immunoproteasomes (**Fig. 2**). Proteasomes were isolated from the EBV-transformed B-cell line LCL-721.174 (constitutive proteasome) and the parental cell line LCL-721 (immunoproteasome, ref. 16) and were used for digestion with a 6-hour incubation period. A combination of Edman sequencing and MALDI-TOF mass spectrometry was subsequently performed to determine the identity and quantity of peptides generated after proteasome digestion. The amount of fragments detected is indicated.

The C-terminal residue Tyr-327 (Y) was generated as the major fragment (314-327) by immunoproteasome cleavage. Furthermore, the complementary fragment 328-336 represented the other major fragment in this digest. Since WT1.314-336 (SEQ ID NO: 6) contains two cysteines at position 325 and 330, which frequently results in disulfide bridging during HPLC separation, an unequivocal assignment of each signal in the mass spectra to a peptide fragment was not possible. Using constitutive proteasomes also a cleavage site C-terminal to Tyr-327 (Y) was demonstrated, since the fragment 328-336 was efficiently generated. The failure to detect the complementary fragment x-327 most likely resulted from the formation of cysteine bridging. In the constitutive proteasome digest also a fragment was found sharing the N-terminus Thr-317 (T) of the WT1.317-327 peptide. No processing of the N-terminus was observed by the immunoproteasome digest. Taken together, these data show the proteasomal generation of epitope WT1.317-327 (SEQ ID NO: 1). While the constitutive proteasome is able to generate both termini of this epitope, the immunoproteasome most likely produces a N-terminally extended precursor. This is in accordance with various reports showing both proteasomal processing of the exact epitopes as well as of N-terminally extended precursors (11,12,22). In contrast to the C-termini, which have to be exactly cleaved by the proteasome, N-termini can be trimmed to their final length by aminopeptidases in the cytoplasm or endo-plasmatic reticulum (23).

### EXAMPLE 2: WT1.317-327 vaccination protocol

For vaccination with WT1.317-327 (SEQ ID NO: 1), the protocol given in (1) describing the vaccination with WT1.126-134 in a HLA-A*0201 positive patient is applied.

An HLA-A*01 positive patient diagnosed with AML receives four biweekly vaccinations with the HLA-A*01-binding WT1 epitope WT1.317-327 in a dose of 0.2 mg admixed with 1 mg keyhole limpet hemocyanin (KLH, Immucothel, biosyn, Germany) as adjuvant *i.d.* and s.c. on day 0. GM-CSF (Leukomax, Essex Pharma, Germany) in a dose of 75 µg per day is injected s.c. at the same site (proximal thigh) as the WT peptide on days -2 to +1. The combination of both adjuvants is chosen due to the immunological efficacy in melanoma peptide vaccination (25). Vaccination cycles can be repeated if necessary.

During the course of therapy, the patient is monitored with respect to common diagnostic parameters such as blood counts and serum creatinine and urea levels. A first bone marrow analysis is performed 4 weeks after the start of vaccination prior to the third vaccination. In addition to marrow blast quantification, WT1 transcripts in bone marrow are monitored with real-time quantitative PCR analysis according to (26) since WT1 levels in bone marrow and peripheral blood have been shown to be a sensitive marker of myeloid leukaemia (27). T-cell response to WT1.317-327 is performed in a peripheral blood using tetramer and intracellular IFNγ staining by flow cytometry (13).

### EXAMPLE 3: Identification of immunogenic T-cell epitopes of WT1

For an identifying of candidate T-cell epitopes having binding motifs for HLA class I alleles, the SYFPEITHI algorithm is used, and 9-, 10-, 11- and 12-mer peptides are selected. The epitope search is preferably restricted to the conserved region of WT1 amounting to at least 90% of the protein. The known HLA2-A2-binding epitope WT1.126-134, for example, is part of this region. The probability for identifying the most useful HLA-binding epitope is about 80% if 2% candidate epitopes with the highest prediction value are screened; this corresponds to about 8 peptides per HLA allele in case of WT1 comprising 450 amino acids.

First, putative epitopes are identified by *ex vivo* screening using lymphocytes of AML patients who frequently show spontaneous immunity against WT1 and proteinase 3. Particularly preferred are AML patients (and also CML patients) who are in complete remission after allogeneic transplantation. T-cell reactivity against candidate epitopes is measured via intracellular accumulation of IFNγ using the FACS analysis technique.

Next, a processing of the peptides is assayed using peptide-specific T-cell lines, and their HLA restricted cytotoxicity for peptide loaded WT1-positive target cells is tested. Specific T-cells are obtained by isolating CD8⁺ T-cells from PBMC using the Mini-MACs-System (Miltenyi, Germany), and 4 x 10⁶ CD8⁺ T-cells are cultured. The CD8- fraction is irradiated, peptide loaded and used for stimulating the CD8⁺ T-cells. Culturing is in the presence of IL-2 and IL-7. Cells are re-stimulated weekly with PBMC that have been loaded with peptide and irradiated. Prior to re-stimulation, the T-cell response is determined in the ELISPOT assay, and in case of sufficient response (at least 1%), a chromium release assay against HLA-A2⁺ WT1-positive and WT1-negative target cells is run.

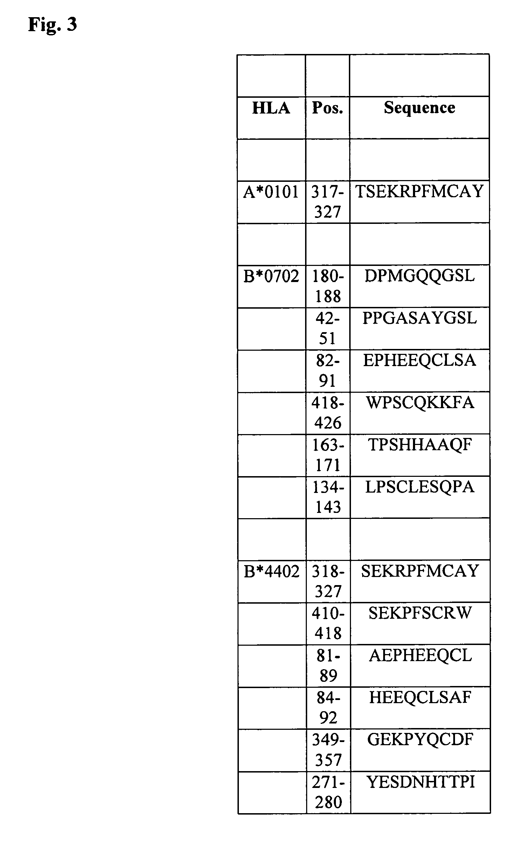

Candidate T-cell epitopes of WT1 are shown in **Fig. 3**.

### References

1. Rosenfeld C, Cheever MA, Gaiger A. WT1 in acute leukemia, chronic myelogenous leukemia and myelodysplastic syndrome: therapeutic potential of WT1 targeted therapies. Leukemia 2003; 17:1301-1312.
2. Mailander V, Scheibenbogen C, Thiel E, Letsch A, Blau IW, Keilholz U. Complete remission in a patient with recurrent acute myeloid leukemia induced by vaccination with WT1 peptide in the absence of hematological or renal toxicity. Leukemia 2004; 18:165-166.
3. Bellantuono I, Gao L, Parry S, et al.. Two distinct HLA-A0201-presented epitopes of the Wilms tumor antigen 1 can function as targets for leukemia-reactive CTL. Blood 2002; 100:3835-3837.
4. Oka Y, Elisseeva OA, Tsuboi A, et al.. Human cytotoxic T-lymphocyte responses specific for peptides of the wild-type Wilms' tumor gene (WT1) product. Immunogenetics 2000; 51:99-107.
5. Azuma T, Makita M, Ninomiya K, et al.. Identification of a novel WT1-derived peptide which induces human leucocyte antigen-A24-restricted anti-leukaemia cytotoxic T lymphocytes. Br J Haematol 2002; 116:601-603.
6. Boon T and Old LJ. Cancer Tumor antigens. Curr Opin Immunol 1997; 9:681-683
7. Scheibenbogen C, Sun Y, Keilholz U, et al.. Identification of known and novel immunogenic T-cell epitopes from tumor antigens recognized by peripheral blood T cells from patients responding to IL-2-based treatment. Int J Cancer 2002; 98:409-414.
8. Hebart H, Daginik S, Stevanovic S, et al.. Sensitive detection of human cytomegalovirus peptide-specific cytotoxic T-lymphocyte responses by interferon-gamma-enzyme-linked immunospot assay and flow cytometry in healthy individuals and in patients after allogeneic stem cell transplantation. Blood 2002; 99:3830-3837
9. Reynolds SR, Celis E, Sette A, et al.. Identification of HLA-A*03, A*11 and B*07-restricted melanoma-associated peptides that are immunogenic in vivo by vaccine-induced immune response (VIIR) analysis. J Immunol Methods 2000; 244:59-67.
10. Provenzano M, Mocellin S, Bettinotti M, et al.. Identification of immune dominant cytomegalovirus epitopes using quantitative real-time polymerase chain reactions to measure interferon-gamma production by peptide-stimulated peripheral blood mononuclear cells. J Immunother 2002; 25:342-351.
11. Kessler JH, Beekman NJ, Bres-Vloemans SA, et al.. Efficient identification of novel HLA-A(*)0201-presented cytotoxic T lymphocyte epitopes in the widely expressed tumor antigen PRAME by proteasome-mediated digestion analysis. J Exp Med 2001; 193:73-88.
12. Lucchiari-Hartz M, van Endert PM, Lauvau G, Maier R, Meyerhans A, Mann D, Eichmann K, Niedermann G. Cytotoxic T lymphocyte epitopes o HIV-1 Nef: Generation of multiple definitive major histocompatibility complex class I ligands by proteasomes. J Exp Med 2000; 191:239-252.
13. Scheibenbogen C, Letsch A, Thiel E, et al.. CD8 T-cell responses to Wilms tumor gene product WT1 and proteinase 3 in patients with acute myeloid leukemia. Blood 2002; 100:2132-2137.
14. Moris A, Teichgräber V, Gauthier L, Bühring HJ, Rammensee HG. Cutting edge: characterization of allorestricted and peptide-selective alloreactive T cells using HLA-tetramer selection. J Immunol 2001; 166:4818-4821.
15. Bodinier M, Peyrat MA, Tournay C, et al.. Efficient detection and immunomagnetic sorting of specific T cells using multimers of MHC class I and peptide with reduced CD8 binding. Nat Med 2000; 6:707-710.
16. Tenzer S, Stoltze L, Schonfisch B, et al.. Quantitative analysis of prion-protein degradation by constitutive and immuno-20S proteasomes indicates differences correlated with disease susceptibility. J Immunol 2004; 172:1083-1091.
17. Rammensee H, Bachmann J, Emmerich NP, Bachor OA, Stevanovic S. SYFPEITHI: database for MHC ligands and peptide motifs. Immunogenetics 1999; 50:213-219.
18. Molldrem JJ, Lee PP, Wang C, et al.. Evidence that specific T lymphocytes may participate in the elimination of chronic myelogenous leukemia. Nat Med 2000; 6:1018-1023.
19. Rezvani K, Grube M, Brenchley JM, et al.. Functional leukemia-associated antigen-specific memory CD8+ T cells exist in healthy individuals and in patients with chronic myelogenous leukaemia before and after stem cell transplantation. Blood 2003; 102:2892-2900.
20. Hilbers U, Ganepola S, Geppert TL, et al.. Induction of an allogeneic CD8+ T cell response against several peptides derived from survivin in patients with hematological disease. Onkologie 2003; 26:P987, 205.
21. Sette A, Sidney J. Nine major HLA class I supertypes account for the vast preponderance of HLA-A and -B polymorphism. Immunogenetics 1999; 50:201-212
22. Mo XY, Cascio P, Lemerise K, Goldberg AL, Rock K. Distinct proteolytic processes generate the C and N termini of MHC class I-binding peptides. J Immunol 1999; 163:5851-5859.
23. Snyder HL, Yewdell JW, Bennink JR. Trimming of antigenic peptides in an early secretory compartment. J Exp Med. 1994; 180:2389-2394.
24. Nussbaum AK, Kuttler C, Hadeler KP, Rammensee HG, Schild H. PAProC: a prediction algorithm for proteasomal cleavages available on the WWW. Immunogenetics 2001; 53:87-94.
25. Scheibenbogen C, Schadendorf D, Bechrakis N, Nagorsen D, Hofmann U, Serve-topoulou F et al. Effects of granulocyte-macrophage colony stimulating factor and foreign helper protein as immunologic adjuvans on the T-cell response to vaccination with tyrosinase peptides. Int J Cancer 2003; 104:188-194.
26. Siehl J, Thiel E, Heufelder K, Snarsk E, Schwartz S, Mailänder V et al. Regulation of Wilm's tumor gene 1 /wt1) expression by the paired box genes PAX2 and PAX8 and by the hematopoietic transcription factor GATA-1 in human acute myeloid leukemias. Br J Hematol 1003; 123:235-242.
27. Cilloni D, Gottardi E, De Micheli D, Serra A, Volpe G, Messa F et al. Quantitative assessment of WT1 expression by real time quantitative PCR may be a useful tool fro monitoring minimal residual disease in acute leukaemia patients. Leukemia 2002; 16:2115-2121.
28. Ysukama M. Immunotherapy for leukaemia targeting the Wilms' tumor gene. Leuk Lymphoma 2000; 42:267-273.
29. Makita M, Hiraki A, Azuma T, Tsuboi A, Oka Y, Sugiyama H, Fujita S et al. Anti-lung cancer effect of WT1-specific cytotoxic T lymphocytes. Clin Cancer Res 2002; 8:2626-2631.
30. Oka Y, Tsuboi A, Elisseeva OA, Udaka K, Sugiyama H. WT1 as a novel target antigen for cancer immunotherapy. Curr Cancer Drug Targets. 2002; 2:45-54.
31. Müller L, Knights A, Pawelec G. Synthetic peptides derived from the Wilm's tumor 1 protein sensitize human T lymphocytes to recognize chronic myelogenous leukaemia cells. Hematol J 2003; 4:57-66.
32. Oka Y, Tsuboi A, Murakam M, Hirai M, Tominaga N, Nakajima H et al. Wilms tumor gene peptide-based immunotherapy for patients with overt leukemia from myelodysplastic syndrome (MDS) ode MDS with myelofibrosis. Int J Hematol 2003; 78:56-61.
33. Oka Y, Tsuboi A, Taguchi T, Osaki T, Kyo T, Nakajima H. Induction of WT1 (Wilms' tumor gene)-specific cytotoxic T lymphocytes by WT1 peptide vaccine ad the resultant cancer regressen. Proc Natl Acad Sci USA 2004; 101:13885-13890.
34. Tsuboi A, Oka Y, Osaki T, Kumagai T, Tachibana I, Hayshi S. WT1 peptide-based immunotherapy for patients with lung cancer: report of two cases. Microbiol Immunol 2004; 48:175-184.
35. Parker KC, Bednarek MA, Coligan JE. Scheme for ranking potential HLA-A2 binding peptides based on independent binding of individual peptide side-chains. J Immunol 1994; 152:163.

## Claims

1. A polypeptide comprising an amino acid sequence as depicted in SEQ ID NO: 1 or a portion or a variant thereof.

2. A polypeptide consisting of an amino acid sequence as depicted in SEQ ID NO: 1 or a portion or a variant thereof.

3. The polypeptide according to claim 1 or 2, wherein the polypeptide is an immunogenic HLA-A*01-binding T-cell epitope of a WT1 protein.

4. A pharmaceutical composition comprising the polypeptide according to any of claims 1-3.

5. The pharmaceutical composition according to claim 4, additionally comprising at least one polypeptide comprising an amino acid sequence or a portion or a variant thereof selected from the group comprising amino acid sequences as depicted in SEQ ID NO: 8-20.

6. A use of the polypeptide according to any of claims 1-3 for the manufacture of a pharmaceutical for the treatment of a disease selected from the group comprising WT1 expressing leukaemia and solid tumours.

7. The use according to claim 6, wherein the leukaemia is acute myeloid leukaemia.

8. An *in vitro* use of the polypeptide according to any of claims 1-3.

9. The *in vitro* use according to claim 8, wherein the polypeptide is used for stimulating and/or expanding T-cells in a sample.

10. The use according to claim 9, wherein the sample is selected from the group comprising bodily fluid and cultured T-cells.

11. An antibody recognizing the polypeptide according to any of claims 1-3.

12. A use of the antibody according to claim 11 for the manufacture of a pharmaceutical for the treatment of a disease selected from the group comprising WT1 expressing leukaemia and solid tumours, preferably acute myeloid leukaemia.

13. An *in vitro* use for identification and/or selection of one or more cells expressing the polypeptide according to any of claims 1-3.

14. The *in vitro* use according to claim 13 for diagnosis and/or monitoring of a disease selected from the group comprising WT1 expressing leukaemia and solid tumours, preferably acute myeloid leukaemia.

15. A method for diagnosis and/or monitoring of a disease selected from the group comprising WT1 expressing leukaemia and solid tumours, preferably acute myeloid leukaemia, using the antibody according to claim 11.

16. The method according to claim 15, comprising the steps of:
(a) providing a sample obtained from a patient suspected to contain the polypeptide according to any of claims 1-3;
(b) contacting said sample with the antibody according to claim 11;
(c) detecting and/or quantifying an interaction between the polypeptide of (a) and the antibody of (b);
(d) comparing the result obtained in (c) with a reference and/or with a result obtained from a reference sample.

17. A polynucleotide encoding an amino acid sequence as depicted in SEQ ID NO: 1 or a portion or a variant thereof.

18. A use of the polynucleotide according to claim 17 for the manufacture of a pharmaceutical for the treatment of a disease selected from the group comprising WT1 expressing leukaemia and solid tumours, preferably acute myeloid leukaemia.

19. A polynucleotide complementary to the polynucleotide according to claim 17.

20. A use of the polynucleotide according to claim 19 for the manufacture of a pharmaceutical for the treatment of a disease selected from the group comprising WT1 expressing leukaemia and solid tumours, preferably acute myeloid leukaemia.

21. An *in vitro* use of the polynucleotide according to claim 19 for diagnosis and/or monitoring of a disease selected from the group comprising WT1 expressing leukaemia and solid tumours, preferably acute myeloid leukaemia.

22. A method for diagnosis and/or monitoring of a disease selected from the group comprising leukaemia and solid tumours, preferably acute myeloid leukaemia, using the polynucleotide according to claim 19.

23. The method according to claim 22, comprising the steps of:
(a) providing a sample obtained from a patient suspected to contain a polynucleotide according to claim 17;
(b) contacting said sample with the polynucleotide according to claim 19;
(c) detecting and/or quantifying an interaction between the polynucleotide of (a) and the antibody of (b);
(d) comparing the result obtained in (c) with a reference and/or with a result obtained from a reference sample.

24. A cytotoxic T-lymphocyte recognizing a polypeptide according to any of claims 1-3.

25. An antigen presenting cell expressing a polypeptide according to any of claims 1-3.

26. A method of identification of an immunogenic T-cell epitope comprising the following steps:
(a) prediction of a potential epitope
(b) screening of said epitope for recognition by T-cells; and
(c) *in vitro* degradation proteasomal cleavage.

27. The method according to claim 26, wherein the T-cell epitope is one of a WT protein.


SEQUENCE LISTING

<110>  Charité; Universitätsmedizin Berlin

<120>  HLA-A*01-binding T-cell epitope of WT1

<130>  FB14366

<160>  20

<170>  PatentIn version 3.2

<210>  1
<211>  11
<212>  PRT
<213>  Artificial

<220>
<223>  T-cell epitope of human WT1, HLA-A*01 WT1.317-327

<400>  1

Thr Ser Glu Lys Arg Pro Phe Met Cys Ala Tyr
1               5                   10


<210>  2
<211>  12
<212>  PRT
<213>  Artificial

<220>
<223>  T-cell epitope of human WT1, HLA-A*01 WT1.37-48

<400>  2

Val Leu Asp Phe Ala Pro Pro Gly Ala Ser Ala Tyr
1               5                   10


<210>  3
<211>  12
<212>  PRT
<213>  Artificial

<220>
<223>  T-cell epitope of human WT1, HLA-A*01 WT1.260-271

<400>  3

Trp Thr Glu Gly Gln Ser Asn His Ser Thr Gly Tyr
1               5                   10

<210> 4
<211> 13
<212> PRT
<213> Artificial

<220>
<223> T-cell epitope of human WT1, HLA-A*01 WT1.137-149

<400> 4

Cys Leu Glu Ser Gln Pro Ala Ile Arg Asn Gln Gly Tyr
1               5                   10


<210> 5
<211> 13
<212> PRT
<213> Artificial

<220>
<223> T-cell epitope of human WT1, HLA-A*01 WT1.188-200

<400> 5

Leu Gly Glu Gln Gln Tyr Ser Val Pro Pro Pro Val Tyr
1               5                   10


<210> 6
<211> 24
<212> PRT
<213> Artificial

<220>
<223> T-cell epitope of human WT1 (precursor), HLA-A*01 WT1.314-336

<400> 6

Ala Ser Glu Thr Ser Glu Lys Arg Pro Phe Met Cys Ala Tyr Pro

1               5                   10                  15

Gly Cys Asn Lys Arg Tyr Phe Lys

                20


<210> 7
<211> 9
<212> PRT
<213> Artificial

<220>
<223> T-cell epitope of HIV gag p17 70-78

<400> 7

Gly Ser Glu Glu Leu Arg Ser Leu Tyr
1               5


<210> 8
<211> 11

<212>  PRT
<213>  Artificial

<220>
<223>  T-cell epitope of human WT1, HLA-A*0101 WT1.317-327

<400>  8

Thr Ser Glu Lys Arg Pro Phe Met Cys Ala Tyr
1               5                   10


<210>  9
<211>  9
<212>  PRT
<213>  Artificial

<220>
<223>  T-cell epitope of human WT1, HLA-B*0702 WT1.180-188

<400>  9

Asp Pro Met Gly Gln Gln Gly Ser Leu
1               5


<210>  10
<211>  10
<212>  PRT
<213>  Artificial

<220>
<223>  T-cell epitope of human WT1, HLA-B*0702 WT1.42-51

<400>  10

Pro Pro Gly Ala Ser Ala Tyr Gly Ser Leu
1               5                   10


<210>  11
<211>  10
<212>  PRT
<213>  Artificial

<220>
<223>  T-cell epitope of human WT1, HLA-B*0702 WT1.82-91

<400>  11

Glu Pro His Glu Glu Gln Cys Leu Ser Ala
1               5                   10


<210>  12
<211>  9
<212>  PRT
<213>  Artificial

<220>
<223>  T-cell epitope of human WT1, HLA-B*0702 WT1.418-426

<400>  12

Trp Pro Ser Cys Gln Lys Lys Phe Ala
1               5

<210>  13
<211>  9
<212>  PRT
<213>  Artificial

<220>
<223>  T-cell epitope of human WT1, HLA-B*0702 WT1.163-171

<400>  13

Thr Pro Ser His His Ala Ala Gln Phe
1               5

<210>  14
<211>  10
<212>  PRT
<213>  Artificial

<220>
<223>  T-cell epitope of human WT1, HLA-B*0702 WT1.134-143

<400>  14

Leu Pro Ser Cys Leu Glu Ser Gln Pro Ala
1               5                   10

<210>  15
<211>  10
<212>  PRT
<213>  Artificial

<220>
<223>  T-cell epitope of human WT1, HLA-B*4402 WT1.318-327

<400>  15

Ser Glu Lys Arg Pro Phe Met Cys Ala Tyr
1               5                   10

<210>  16
<211>  9
<212>  PRT
<213>  Artificial

<220>
<223>  T-cell epitope of human WT1, HLA-B*4402 WT1.410-418

<400>  16

Ser Glu Lys Pro Phe Ser Cys Arg Trp
1               5

<210>  17
<211>  9

<212>  PRT
<213>  Artificial

<220>
<223>  T-cell epitope of human WT1, HLA-B*4402 WT1.81-89

<400>  17

Ala Glu Pro His Glu Glu Gln Cys Leu
1               5


<210>  18
<211>  9
<212>  PRT
<213>  Artificial

<220>
<223>  T-cell epitope of human WT1, HLA-B*4402 WT1.84-92

<400>  18

His Glu Glu Gln Cys Leu Ser Ala Phe
1               5


<210>  19
<211>  9
<212>  PRT
<213>  Artificial

<220>
<223>  T-cell epitope of human WT1, HLA-B*4402 WT1.349-357

<400>  19

Gly Glu Lys Pro Tyr Gln Cys Asp Phe
1               5


<210>  20
<211>  10
<212>  PRT
<213>  Artificial

<220>
<223>  T-cell epitope of human WT1, HLA-B*4402 WT1.271-280

<400>  20

Tyr Glu Ser Asp Asn His Thr Thr Pro Ile
1               5                   10